# FASCICULE DE BREVET EUROPEEN

(11) **EP 1 237 507 B1**
(45) Date de publication et mention de la délivrance du brevet: **19.07.2006**
(21) Numéro de dépôt: 00979720.0
(22) Date de dépôt: 10.11.2000
(51) Int. Cl.: A61F 2/16

(54) **IMPLANT INTRAOCULAIRE PRECRISTALLINIEN**
PHAKISCHE INTRAOKULARLINSE
PRE-CRYSTALLINE INTRAOCULAR IMPLANT

(30) Priorité: 10.11.1999 FR 9914156
(43) Date de publication de la demande: 11.09.2002
(73) Titulaire: Ioltechnologie-Production, 17000 La Rochelle (FR)
(72) Inventeur: LORENZO, Georges, AR-3000 Santa Fe (AR); PLATON, Olivier, 13190 Allauch (FR)
(74) Mandataire: Santarelli
(86) Numéro de dépôt international: PCT/FR2000/003156
(87) Numéro de publication internationale: WO 2001/034066

(56) Documents cités:
- EP-A- 0 592 813
- WO-A-97/30657
- WO-A-98/17205
- DE-C- 4 211 265
- GB-A- 2 153 688
- US-A- 4 769 035
- US-A- 5 913 898

## Description

La présente invention conceme un implant intraoculaire précristallinien.

Elle vise de manière générale les implants intraoculaires de chambre postérieure d'un oeil phaque, notamment pour la correction des amétropies d'un sujet jeune à cristallin clair.

Un tel implant intraoculaire est adapté à être implanté entre la face antérieure du cristallin et l'iris.

L'exiguïté et l'environnement de l'espace entre l'iris et le cristallin posent des problèmes pour la réussite de ce type d'implant. En effet, un contact trop fort avec la face postérieure de l'iris peut induire sa dépigmentation et le frottement avec la cristalloïde peut générer une cataracte du cristallin.

De même, un tel implant est soumis aux mouvements antéro-postérieurs du centre du cristallin lors de l'accommodation pour la vision de près, ce qui pose des problèmes de stabilité et de centrage d'un tel implant.

La surface postérieure de l'optique des implants précristalliniens est sphérique, présente un rayon déterminé, typiquement 10,5 mm. Un tel choix est avantageux du point de vue de la fabrication mais à l'état de repos, la face antérieure du cristallin n'a pas une seule surface sphérique mais en réalité une surface asphérique, avec une infinité de surfaces sphériques successives.

On comprendra qu'un implant dont la surface sphérique postérieure a un seul rayon, voire deux, ne va pas pouvoir épouser la surface antérieure du cristallin.

Dans le document WO-A-98/17205, il est décrit un implant intraoculaire précristallinien comprenant une partie optique et une partie haptique. La face postérieure de l'implant comporte une première portion sphérique centrale de premier rayon et une deuxième portion de surface de second rayon plus grand que le premier rayon.

De même, les Implants précristalliniens dont la surface postérieure de l'optique a un seul, voire deux rayons de courbure, sont plus ou moins bien positionnés par rapport à la surface antérieure du cristallin. Il en résulte une certaine instabilité du positionnement de l'implant par rapport au cristallin.

En outre, la plupart des implants précrlstalliniens sont censés s'écarter de la face antérieure du cristallin et donc éviter tout contact avec elle.

Le but de la présente invention est de proposer un implant intraoculaire précristallinien qui permet à l'implant d'épouser étroitement la face antérieure du cristallin et qui est d'un encombrement minimal.

A cet effet, la présente invention vise un implant intraoculaire précristallinien adapté à être implanté entre la face antérieure du cristallin et l'iris, comprenant une partie optique et une partie haptique, la partie optique étant formée sur un corps central de l'implant sans gêner l'accommodation.

Selon une caractéristique de l'invention, la face postérieure de la lentille comporte une première portion sphérique centrale d'un premier rayon et une deuxième portion sphérique annulaire d'un second rayon plus grand que le premier rayon, la troisième portion sphérique concentrique entourant la deuxième portion d'un troisième rayon plus grand que le deuxième rayon.

Selon un mode de réalisation préféré de cet aspect de l'invention, le diamètre exteme de la couronne périphérique est inférieur ou égal au diamètre de la face antérieure du cristallin et n'est donc pas en appui dans le sulcus. Dans ce cas, le centrage et la stabilité du positionnement sont assurés par la complémentarité des surfaces sphériques de la face postérieure du corps et les zones correspondantes de la face antérieure du cristallin. Dans ce mode de réalisation, la partie haptique de la troisième portion de surface sphérique concentrique est adaptée à la partie correspondante du cristallin à l'état de repos. De grandes ouvertures dans la partie haptique servent à maintenir le métabolisme du cristallin.

Ce mode de réalisation préféré présente un avantage important lors de l'Implantation. Cet implant doit être relâché dans la chambre postérieure en face du cristallin sans avoir à se préoccuper de l'emplacement de la partie périphérique dans le sulcus ciliaire, qui est hors du champ de vision du chirurgien. En outre, un tel implant peut être introduit par une incision cornéenne ou scléro-cornéenne de 2 mm auto-étanche, étant donné la faible épaisseur de l'Implant et le diamètre réduit d'environ 10 mm. Enfin, dans ce dernier mode de réalisation, la surface postérieure de l'implant épouse la face antérieure du cristallin à l'état de repos sur trois zones différentes de rayon allant du plus petit au centre au plus grand à la périphérie.

Grâce à la configuration de la surface postérieure du corps central, celui-ci est parfaitement adapté à la convexité de la surface de la face antérieure du cristallin, de manière à augmenter la surface de contact réelle entre la face postérieure du corps central et le cristallin et réduire au maximum l'encombrement,

Avec des implants selon l'invention, le ou les rayons de courbure de la face antérieure du corps central sont calculés en fonction de l'amétropie à corriger.

Selon une forme de réalisation de l'invention, c'est l'ensemble du corps qui constitue la partie optique, par exemple monofocale, et la face antérieure du corps comporte une surface centrale sphérique d'un premier rayon et une surface annulaire sphérique d'un second rayon. Une telle forme de réalisation est particulièrement adaptée à une optique ménisquée convergente pour la correction de l'hypermétropie.

Selon encore une autre forme de réalisation, c'est seulement la partie centrale du corps qui définit la partie optique, le corps ayant un anneau non réfractif entourant cette partie centrale. Avec un tel corps, la surface antérieure peut avantageusement être sphérique avec un seul rayon de courbure pour le cas d'une optique divergente pour la correction de la myopie (à savoir optique ménisquée divergente ou concave).

Malgré le contact étroit obtenu entre la face postérieure du corps central de l'implant et la face antérieure du cristallin au repos, la circulation de l'humeur aqueuse entre ces deux surfaces reste assurée, notamment du fait des mouvements antéro-postérieurs du centre du cristallin lors de l'accommodation. Pour l'accommodation, il y a des modifications du rayon de courbure de la zone centrale de la face antérieure du cristallin et avec aspiration de l'humeur aqueuse dans l'espace ainsi formé entre le corps et le cristallin .

Afin de permettre l'insertion de l'Implant par une incision de taille réduite, le matériau constitutif de l'implant est souple, avec un taux d'hydrophilie important et un indice de réfraction élevé. Un tel implant peut avoir une épaisseur minimale proche de 30 µm au centre pour des implants de dioptries négatives destinés aux myopes et de 30 µm au bord de l'optique pour des implants de dioptries positives destinés aux hypermétropes. Pour que le contact avec l'iris et la cristalloïde soit sans incidence physiologique l'un sur l'autre, la surface de l'implant sera la plus lisse possible, avec un polissage atteignant 2 à 3 nm.

Selon une caractéristique préférée de l'invention, la partie haptique comporte une couronne périphérique et des bras reliant le bord périphérique du corps central à la couronne périphérique.

Selon une autre caractéristique préférée de l'invention, la partie haptique comporte des ouvertures de grandes dimensions, délimitées par une paire de bras, le bord du corps et la couronne périphérique. Ces ouvertures de la partie haptique de l'implant intraoculaire, situées à la périphérie du cristallin, facilitent un bon contact de l'humeur aqueuse avec la face antérieure du cristallin dans la région équatoriale, malgré la présence de l'implant intraoculaire précristallinien sur cette face antérieure du cristallin.

En effet, les zones métaboliquement actives du cristallin sont situées à la face antérieure du cristallin, et plus particulièrement à l'équateur de celui-ci, où il existe une multiplication cellulaire et où se produit la synthèse des fibres cristalliniennes.

D'autres particularités et avantages de l'invention apparaîtront encore dans la description ci-après.

Aux dessins annexés, donnés à titre d'exemples non limitatifs :
- la figure 1 est une vue de face d'un implant intraoculaire précristallinien conforme à un mode de réalisation préféré de l'invention ;
- la figure 2 est une vue en coupe transversale de l'implant intraoculaire précristallinien illustré à la figure 1 ;
- la figure 3 est une vue en coupe de l'implant intraoculaire précristallinien de la figure 1, en position dans la chambre postérieure de l'oeil ;
- la figure 4 est une vue en coupe transversale de l'Implant Intraoculaire précristallinien, selon une autre forme de réalisation préférée ;
- la figure 5 est une vue en coupe de l'Implant intraoculaire précristallinien, de la figure 4, en position dans la chambre postérieure de l'oeil ;
- la figure 6 est également une vue en coupe transversale de l'implant intraoculaire précristallinien selon une autre forme de réalisation préférée ;
- la figure 7 est une vue en coupe de l'Implant intraoculaire précristallinien, selon la figure 6, en position dans la chambre postérieure de l'oeil ;
- la figure 8 est une vue en section partielle à plus grande échelle pour illustrer des rayons de courbure de trois portions sphériques concentriques, sur la face postérieure de l'implant des figures 2, 4 et 6.

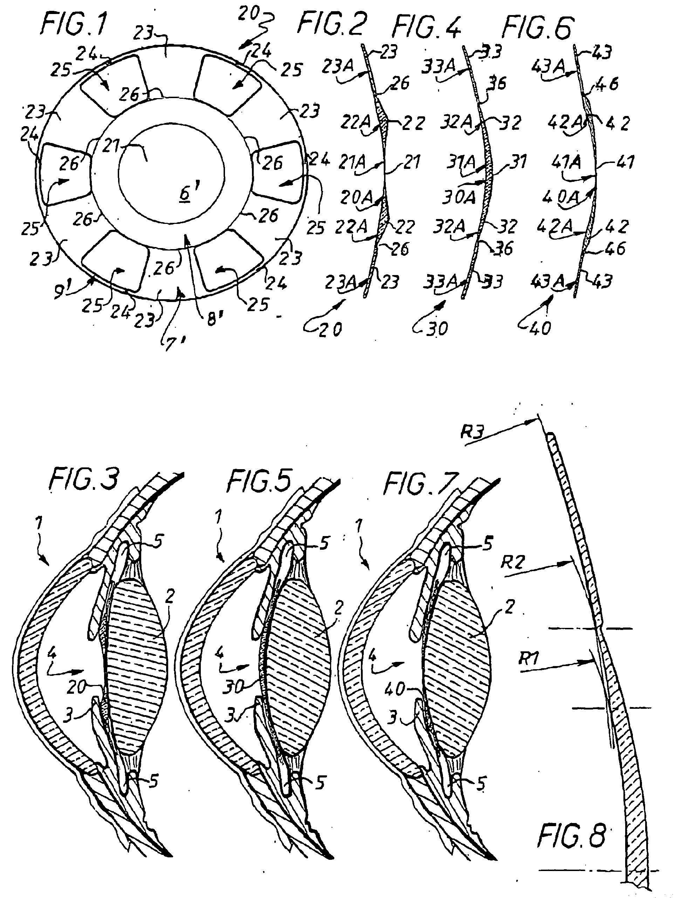

Un mode de réalisation conforme à l'invention va maintenant être décrit en référence aux figures 1 à 8.

Dans ce mode de réalisation, la couronne périphérique 24 n'est pas en appui dans le sulcus ciliaire mais est maintenue en position sur la face antérieure du cristallin par complémentarité de forme entre la surface postérieure de l'implant qui épouse étroitement la face antérieure du cristallin (et éventuellement par le contact avec la surface postérieure de l'iris), à la manière d'une lentille de contact placée entre la cornée et la paupière.

Comme illustré aux figures 3, 5 et 7, un implant intraoculaire 20, 30, 40 est également adapté à être implanté entre la face antérieure du cristallin 2 et l'iris 3, dans un oeil phaque 1. Il comporte une partie optique 6' et une partie haptique 7', son corps central 8', est entouré d'une structure de support 9'.

Sur le corps central 8' est également formée une zone optique centrale 21, laquelle peut être adaptée à corriger la myopie, tel qu'illustré dans les figures 2, 3, 6 et 7.

De manière générale, cette partie optique, par la géométrie de sa surface antérieure est adaptée à corriger toute autre amétropie d'un oeil phaque, et notamment l'hypermétrople, tel qu'illustré sur les figures 4 et 5.

Dans ce cas, la partie optique 6' est constituée par une lentille ménisquée convergente, telle que couramment utilisée pour corriger certaines hypermétropies.

La géométrie de la surface antérieure de la partie optique peut être adaptée à corriger la myopie de l'oeil phake, tel qu'illustré sur la figure 6. Cet exemple correspond à des myopies de 0 à -12 dioptries. Dans ce cas, la partie optique 6' est constituée par une lentille ménisquée divergente. Pour les plus fortes myopies (au-delà de -12 dioptries dans le présent exemple), l'optique est biconcave, comme illustré à la figure 2.

De manière tout à fait générale, la partie optique de l'implant peut être adaptée à corriger, outre les amétropies habituelles, également la presbytie par des techniques d'implants bifocaux, voire multifocaux bien connues dans ce domaine, tout en respectant les portions de surface sphérique sur la face postérieure du corps.

La partie haptique 7' de cet implant intraoculaire 20, et 40 comporte un anneau non réfractif 22 et 42 sur le corps 8' entourant la zone optique 21, une couronne périphérique 24, 44 et des bras 23, 43 reliant l'anneau non réfractif 22, 42 du corps à la couronne périphérique 24. En revanche, dans l'Implant intraoculaire 30, la partie haptique 7' comporte une couronne périphérique 34 et des bras 33 reliant le corps à la couronne périphérique 24.

De préférence, afin d'améliorer la circulation de l'humeur aqueuse, les ouvertures 25 de la partie haptique s'étendent sur un secteur angulaire total supérieur ou égal à 180°.

Dans cet exemple, la partie haptique comporte des bras radiaux 23, 33, 43 en forme de secteur angulaire délimitant deux à deux des ouvertures 25. Un des bords radiaux d'une des ouvertures peut comporter une encoche en tant que repère de la face avant de l'implant.

Ici, chaque bras radial 23, 33, 43 et chaque ouverture 25 s'étendent sur des secteurs angulaires égaux.

En outre, afin d'améliorer la souplesse de cet implant, la partie haptique comporte également des portions amincies 26, 36, 46 qui forment chamières à la jonction des bras 23, 33, 43 et de l'anneau non réfractif 22 sur le corps central 8'.

Dans ce mode de réalisation, les dimensions hors tout de cet implant intraoculaire 20, 30, 40 sont telles que la couronne périphérique 24 de la partie haptique ne vient pas en appui dans le sulcus ciliaire 5 de l'oeil, comme Illustré aux figures 3, 5 et 7, en pratique ne déborde pas de la périphérie de la face antérieure du cristallin.

Dans ce mode de réalisation, cet implant intraoculaire précristallinien 20, 30, 40 est simplement maintenu entre la face antérieure du cristallin et le contact Inévitable avec le rideau irien, s'agissant d'une partie optique biconcave (Fig. 3), ou ménisquée convergente (Fig 5) ou ménisquée divergente (Fig 7).

Afin de rester centré dans cette chambre postérieure 5, l'implant intraoculaire précristallinien est adapté à adhérer spontanément sur la face

A cet effet, la face postérieure 20A, 30A, 40A de l'implant 20, 30, 40 comporte trois portions de surface concentriques 21A, 22A, 23A, 31A, 32A, 33A, 41A. 42A, 43A dont les rayons de courbure sont sensiblement égaux à la courbure de la face antérieure correspondante du cristallin 2 au repos.

Les deux premières portions 21A, 22A, 31A, 32A, 41A et 42A de surface concentriques correspondent, comme précédemment, à la zone optique centrale 21, 31, 41 et à l'anneau 22, 32, 42 de l'implant 20, 30, 40.

La troisième portion 23A, 33A, 43A de surface concentrique correspond quant à elle aux bras 23, 33, 43 et à la couronne périphérique 24 de la partie haptique de l'implant intraoculaire 20, 30, 40.

Ce triple rayon de courbure de la face postérieure 20A, 30A. 40A de l'implant 20, 30, 40 permet à cet implant d'épouser étroitement la face antérieure du cristallin 2 au repos.

La présence de ce troisième rayon de courbure (R3) sur la face postérieure 20A 30A, 40A de l'implant 20, 30, 40 permet de se passer d'une couronne périphérique haptique venant en appui dans le sulcus ciliaire 5.

Dans un mode de réalisation pratique, donné ici à titre d'exemple non limilltif, la zone optique centrale 21, 31, 41 a un diamètre sensiblement égal à 4 mm, le rayon de courbure de sa face postérieure de la première portion 21A, 31A, 41A de la surface sphérique étant entre 8 et 10,5 mm et de préférence égal à 9,5 mm.

L'anneau 22, 32, 42 présente un diamètre sensiblement égal à 6 mm, le rayon de courbure de sa face postérieure étant entre 12 et 14 mm et de préférence sensiblement égal à 13 mm.

Enfin, les bras radiaux 23, 33, 43 au nombre de six dans cet exemple, ainsi que la couronne périphérique 24 sont inscrits dans un cercle de diamètre d'au moins 10 mm, et de préférence 11 mm. Cette dimension doit être inférieure ou égale au diamètre de la face antérieure du cristallin. Le rayon de courbure de la face antérieure et postérieure de cette portion de l'implant, constituées des bras radiaux 23, 33, 43 et de la couronne périphérique 24 est entre 17 et 25 mm et de préférence sensiblement égal à 17 mm. périphérique 24 est entre 17 et 25 mm et de préférence sensiblement égal à 17 mm.

Comme précédemment, on constate que les rayons R1, R2, R3 de courbure de la face postérieure (voir Figure 8) de chacun des implants 20, 30, 40 va en augmentant du centre vers la périphdrie de l'implant, afin de s'adapter au mieux à la topographie de la chambre postérieure définie par la face antérieure du cristallin 2, en arrière, et de la face postérieure de l'iris en avant.

Dans les trois cas, cet implant se loge au mieux dans la forme de la chambre postérieure 4 sans la modifier, c'est-à-dire sans frotter sur l'iris en avant, ni pousser sur le cristallin en arrière, et ce avec un encombrement minimum.

Il est en outre important de choisir un matériau suffisamment souple, d'hydrophilie maximale avec un indice de réfraction élevé pour pouvoir être introduit par une incision très courte avec un traumatisme opératoire minimal.

Il va de soi que le matériau doit en outre être stable dans le temps, non irritant et bien entendu non cataractogène.

Afin que la présence de l'implant perturbe le moins possible le cristallin, ce dernier doit présenter une épaisseur minimale proche de 30 µm au centre pour l'implant destiné à la correction de la myopie et 30 µm au bord du corps pour l'implant destiné à la correction de l'hypermétropie.

En outre, il doit présenter une surface la plus lisse possible, grâce à un polissage atteignant 2 à 3 nm pour éviter la dispersion pigmentaire à long terme par frottement contre la face postérieure de l'iris.

En particulier, les zones métaboliquement actives étant uniquement la face antérieure du cristallin, où existent des cellules épithéliales, et particulièrement l'équateur du cristallin, où il existe une multiplication cellulaire et où se produit la synthèse des fibres cristalliniennes constituant la lentille, il suffit de respecter l'équeteur du cristallin et d'éviter les traumatismes sur la face antérieure du cristallin et la face postérieure de l'iris. En outre, le métabolisme énergétique du cristallin est diversifié, et notamment, ne fait que peu Intervenir l'apport en oxygène provenant de l'humeur aqueuse.

Il n'y donc pas de risque que le contact entre l'implant et la face antérieure du cristallin puisse s'avérer cataractogène, surtout en tenant compte de l'extrême finesse de cet implant et de son caractère hydrophile.

En outre, malgré le contact étroit obtenu entre l'implant et la face antérieure du cristallin au repos, une circulation d'humeur aqueuse entre ces deux surfaces reste possible, du fait des mouvements antério-postérieures du centre du cristallin lors de l'accommodation qui produit une aspiration l'humeur aqueuse.

On obtient ainsi grâce à l'invention, un implant intraoculaire précristallinien qui permet de respecter au mieux l'anatomie et la physiologie de la chambre postérieure de l'oeil.

Bien entendu, de nombreuses modifications pourraient être apportées aux exemples de réalisation décrits ci-dessus sans sortir du cadre de l'invention. En particulier, l'implant intraoculaire précristallinien selon l'invention peut être multlfocal pour la correction de la presbytie. Dans ce cas, à titre d'exemple, la face antérieure de l'implant pourrait comporter, selon une technique connue, une zone optique centrale et une pluralité de zones optiques annulaires concentriques, de rayon de courbure approprié.

Ainsi, la largeur des bras de la partie haptique pourrait ne pas être constante, mais au contraire différente d'un bras à l'autre. Il en est de même pour l'haptique en général.

## Revendications

1. Implant intraoculaire précristallinien adapté à être implanté entre la face antérieure du cristallin (2) et l'iris, comprenant une partie optique (6') et une partie haptique (7'), la partie optique étant formée sur un corps central (8'), la face postérieure de l'implant intraoculaire ayant une première portion sphérique centrale (21A, 31A, 41A) d'un premier rayon (R1) et une deuxième portion de surface sphérique annulaire (22A, 32A, 42A) d'un second rayon (R2) plus grand que le premier rayon, **caractérisé en ce qu'**une troisième portion sphérique concentrique entourant la deuxième portion d'un troisième rayon (R3) plus grand que le deuxième rayon (R2).

2. Implant intraoculaire précristallinien selon la revendication 1, **caractérisé en ce que** les rayons de courbure (R1, R2, R3) desdites portions de surface sont sensiblement égaux aux rayons de courbure correspondants de la face antérieure du cristallin (2) au repos.

3. Implant Intraoculaire précristallinien selon la revendication 1 ou 2, **caractérisé en ce que** l'ensemble du corps (8') constitue la partie optique (6') du type monofocal, et que la face antérieure du corps comporte une surface centrale sphérique d'un premier rayon et une surface annulaire d'un second rayon.

4. Implant intraoculalre précristallinien selon la revendication 1 ou 2, **caractérisé en ce que** la partie optique (6') comprend seulement une zone centrale (21, 41) du corps, le corps ayant une couronne haptique (22, 42) entourant la partie optique.

5. Implant intraoculaire précristellinien selon l'une des revendications 1 à 4, **caractérisé en ce que** la partie haptique (7') comporte une couronne périphérique (24) et des bras (23, 33, 43) reliant le bord périphérique du corps (8') et la couronne périphérique (24).

6. Implant intraoculaire précriatallinien selon la revendication 5, **caractérisé en ce que** la partie haptique (7') comporte des ouvertures (25) de grande dimension, délimitées par une paire de bras (23, 33, 43), le bord périphérique du corps (8') et la couronne périphérique (24).

7. Implant intraoculaire précristallinien selon la revendication 5 ou 6, **caractérisé en ce que** la partie haptique (7') comporte des ouvertures (25) s'étendant sur un secteur angulaire total supérieur ou égal à 180°.

8. Implant intraoculaire précristallinien selon l'une des revendications 5 à 7, **caractérisé en ce que** la partie haptique (7') comporte au moins quatre bras radiaux (23. 33, 43) de même largeur.

9. Implant intraoculaire précristallinien selon l'une des revendications 5 à 8, **caractérisé en ce que** la partie haptique (7') comporte des bras radiaux (23, 33, 43) en forme de secteur angulaire délimitant des ouvertures (35).

10. Implant intraoculaire précristallinien selon l'une des revendications 1 à 9, **caractérisé en ce que** la face postérieure de l'implant (21A, 31A, 41A) est étroitement complémentaire à la face antérieure du cristallin (2) au repos, de sorte qu'il est apte à adhérer spontanément à la face antérieure du cristallin.

11. Implant intraoculaire précristallinien selon la revendication 10, **caractérisé en ce que** la troisième portion de surface sphérique concentrique correspondant aux bras (23, 33, 43) et à la couronne périphérique (24) ayant un rayon de courbure (R3) sensiblement égal au rayon de courbure au niveau de la région équatoriale de la face antérieure du cristallin (2) au repos, le diamètre de la couronne périphérique (24) étant adapté au diamètre cristallinien.

12. Implant intraoculaire précristallinien selon l'une quelconque des revendications précédentes, destiné à la correction de la myopie, **caractérisé en ce que** l'implant présente une épaisseur minimale proche de 30 µm au centre.

13. Implant intraoculaire précristallinien selon l'une quelconque des revendications 1 à 11, destiné à la correction de l'hypermétropie, **caractérisé en ce que** l'implant présente une épaisseur minimale proche de 30 µm au bord du corps.

## Claims

1. A precrystalline intraocular implant adapted to be implanted between the anterior face of the crystalline lens (2) and the iris, the implant comprising an optical part (6') formed on a central body (8') and a haptic part (7'), the posterior face of the intraocular implant including a central spherical first portion (21A, 31A, 41A) having a first radius (R1), an annular spherical second surface portion (22A, 32A, 42A) having a second radius (R2) greater than the first radius, **characterised in that** a concentric spherical third portion surrounds the second portion and has a third radius (R3) greater than the second radius (R2).

2. A precrystalline intraocular implant according to claim 1, **characterised in that** the radii of curvature (R1, R2, R3) of said surface portions are substantially equal to those of the anterior face of the crystalline lens (2) when relaxed.

3. A precrystalline intraocular implant according to claim 1 or claim 2, **characterised in that** the whole of the body (8') constitutes a monofocal optical part (6') and the anterior face of the body includes a spherical central surface having a first radius and an annular surface having a second radius.

4. A precrystalline intraocular implant according to claim 1 or claim 2, **characterised in that** the optical part (6') comprises only a central area (21, 41) of the body, which has a haptic ring (22, 42) surrounding the optical part.

5. A precrystalline intraocular implant according to any of claims 1 to 4, **characterised in that** the haptic part (7') includes a peripheral ring (24) and arms (23, 33, 43) connecting the peripheral edge of the body (8') and the peripheral ring (24).

6. A precrystalline intraocular implant according to claim 5, **characterised in that** the haptic part (7') includes large apertures (25) delimited by a pair of arms (23, 33, 43), the peripheral edge of the body (8'), and the peripheral ring (24).

7. A precrystalline intraocular implant according to claim 5 or claim 6, **characterised in that** the haptic part (7') includes apertures (25) extending over a total angular sector greater than or equal to 180°.

8. A precrystalline intraocular implant according to one of claims 5 to 7, **characterised in that** the haptic part (7') has at least four radial arms (23, 33, 43) of the same width.

9. A precrystalline intraocular implant according to one of claims 5 to 8, **characterised in that** the haptic part (7') has radial arms (23, 33, 43) in the shape of an angular sector delimiting apertures (35).

10. A precrystalline intraocular implant according to one of claims 1 to 9, **characterised in that** the posterior face of the implant (21A, 31A, 41A) is closely complementary to the anterior face of the relaxed crystalline lens (2), in such a way that it is able to adhere spontaneously to the anterior face of the crystalline lens.

11. A precrystalline intraocular implant according to claim 10, **characterised in that** the third concentric spherical surface portion corresponding to the arms (23, 33, 43) and to the peripheral ring (24) has a radius of curvature (R3) substantially equal to the radius of curvature at the equatorial region of the anterior face of the relaxed crystalline lens (2), the diameter of the peripheral ring (24) being matched to the diameter of the crystalline lens.

12. A precrystalline intraocular implant according to any preceding claim, intended for correcting myopia, which implant is **characterised in that** it has a minimum thickness of approximately 30 µm at its centre.

13. A precrystalline intraocular implant according to any of claims 1 to 11, intended for correcting hypermetropia, which implant is **characterised in that** it has a minimum thickness of approximately 30 µm at the edge of the body.

## Patentansprüche

1. Vor der Augenlinse angeordnetes Intraokularimplantat, das so beschaffen ist, dass es zwischen die Vorderseite der Augenlinse (2) und die Iris implantiert werden kann, und einen optischen Abschnitt (6') und einen haptischen Abschnitt (7') umfasst, wobei der optische Abschnitt auf einem Mittelkörper (8') ausgebildet ist und die Rückseite des Intraokularimplantats einen ersten mittigen sphärischen Abschnitt (21A, 31A, 41A) mit einem ersten Radius (R1) und einen zweiten ringförmigen Abschnitt (22A, 32A, 42A) mit sphärischer Oberfläche mit einem zweiten Radius (R2), der größer als der erste Radius ist, besitzt, **dadurch gekennzeichnet, dass** ein dritter, konzentrischer sphärischer Abschnitt den zweiten Abschnitt umgibt und einen dritten Radius (R3) besitzt, der größer als der zweite Radius (R2) ist.

2. Vor der Augenlinse angeordnetes Intraokularimplantat nach Anspruch 1, **dadurch gekennzeichnet, dass** die Krümmungsradien (R1, R2, R3) der Oberflächenabschnitte im Wesentlichen gleich den entsprechenden Krümmungsradien der Vorderseite der in der Ruhestellung befindlichen Augenlinse (2) sind.

3. Vor der Augenlinse angeordnetes Intraokularimplantat nach Anspruch 1 oder 2, **dadurch gekennzeichnet, dass** der gesamte Körper (8') den optischen Abschnitt (6') des monofokalen Typs bildet und dass die Vorderseite des Körpers eine sphärische mittige Oberfläche mit einem ersten Radius und eine ringförmige Oberfläche mit einem zweiten Radius aufweist.

4. Vor der Augenlinse angeordnetes Intraokularimplantat nach Anspruch 1 oder 2, **dadurch gekennzeichnet, dass** der optische Abschnitt (6') nur eine mittige Zone (21, 41) des Körpers umfasst, wobei der Körper einen den optischen Abschnitt umgebenden haptischen Kranz (22, 42) besitzt.

5. Vor der Augenlinse angeordnetes Intraokularimplantat nach einem der Ansprüche 1 bis 4, **dadurch gekennzeichnet, dass** der haptische Abschnitt (7') einen Umfangskranz (24) sowie Arme (23, 33, 43), die den Umfangsrand des Körpers (8') mit dem Umfangskranz (24) verbinden, umfasst.

6. Vor der Augenlinse angeordnetes Intraokularimplantat nach Anspruch 5, **dadurch gekennzeichnet, dass** der haptische Abschnitt (7') groß dimensionierte Öffnungen (25) aufweist, die durch ein Paar Arme (23, 33, 43), den Umfangsrand des Körpers (8') und den Umfangskranz (24) begrenzt sind.

7. Vor der Augenlinse angeordnetes Intraokularimplantat nach Anspruch 5 oder 6, **dadurch gekennzeichnet, dass** der haptische Abschnitt (7') Öffnungen (25) aufweist, die sich über einen gesamten Winkelsektor erstrecken, der größer oder gleich 180° ist.

8. Vor der Augenlinse angeordnetes Intraokularimplantat nach einem der Ansprüche 5 bis 7, **dadurch gekennzeichnet, dass** der haptische Abschnitt (7') wenigstens vier radiale Arme (23, 33, 43) gleicher Breite umfasst.

9. Vor der Augenlinse angeordnetes Intraokularimplantat nach einem der Ansprüche 5 bis 8, **dadurch gekennzeichnet, dass** der haptische Abschnitt (7') radiale Arme (23, 33, 43) in Form eines Winkelsektors, der die Öffnungen (35) begrenzt, umfasst.

10. Vor der Augenlinse angeordnetes Intraokularimplantat nach einem der Ansprüche 1 bis 9, **dadurch gekennzeichnet, dass** die Rückseite des Implantats (21A, 31A, 41A) in der Ruhestellung der Augenlinse (2) zu deren Vorderseite genau komplementär ist, so dass sie an der Vorderseite der Augenlinse spontan anhaften kann.

11. Vor der Augenlinse angeordnetes Intraokularimplantat nach Anspruch 10, **dadurch gekennzeichnet, dass** der dritte Abschnitt mit konzentrischer sphärischer Oberfläche, der den Armen (23, 33, 43) und dem Umfangskranz (24) entspricht, einen Krümmungsradius (R3) besitzt, der im Wesentlichen gleich dem Krümmungsradius auf Höhe der Äquatorialbereichs der Vorderseite der in der Ruhestellung befindlichen Augenlinse (2) ist, wobei der Durchmesser des Umfangskranzes (24) an den Augenlinsendurchmesser angepasst ist.

12. Vor der Augenlinse angeordnetes Intraokularimplantat nach einem der vorhergehenden Ansprüche, das für die Korrektur der Myopie vorgesehen ist, **dadurch gekennzeichnet, dass** das Implantat im Zentrum eine minimale Dicke von etwa 30 µm aufweist.

13. Vor der Augenlinse angeordnetes Intraokularimplantat nach einem der Ansprüche 1 bis 11, das für die Korrektur der Hypermetropie bestimmt ist, **dadurch gekennzeichnet, dass** das Implantat am Rand des Körpers eine minimale Dicke von etwa 30 µm aufweist.
